# EUROPEAN PATENT APPLICATION

(11) **EP 4 310 181 A1**
(43) Date of publication of application: **24.01.2024**
(21) Application number: 21930969.7
(22) Date of filing: 12.04.2021
(51) Int. Cl.: C12N 11/084, C12N 9/02, C12N 9/06, C12N 9/10

(54) **ENZYME IMMOBILIZATION CARRIER AND PREPARATION METHOD THEREFOR, IMMOBILIZED ENZYME AND PREPARATION METHOD THEREFOR**

(30) Priority: 18.03.2021 CN 202110288881
(71) Applicant: Asymchem Laboratories (Tianjin) Co., Ltd., Tianjin 300457 (CN)
(72) Inventor: HONG, Hao, Morrisville, North Carolina 27560 (US); XIAO, Yi, Tianjin 300457 (CN); ZHANG, Na, Tianjin 300457 (CN); LIN, Han, Tianjin 300457 (CN); PAN, Long, Tianjin 300457 (CN); VYASA, Williams, Morrisville, North Carolina 27560 (US); MA, Liteng, Tianjin 300457 (CN); CUI, Yuxia, Tianjin 300457 (CN); GAO, Yanyan, Tianjin 300457 (CN)
(74) Representative: Studio Torta S.p.A.
(86) International application number: PCT/CN2021/086365
(87) International publication number: WO 2022/193381

(57) **Abstract**

The application provides an enzyme immobilization carrier and a preparation method thereof, an immobilized enzyme and a preparation method thereof. The above enzyme immobilization carrier is obtained by an amino modification or a cyanuric chloride modification of super-crosslinked polyvinyl alcohol. The use of the enzyme immobilization carrier provided by the application may effectively improve the stability and reusability of the immobilized enzyme. Moreover, due to the use of the form of enzyme covalent linkage, compared with an embedding method, the preparation method is no need for chemical reagent immersion and the like, it is beneficial to maintain the own activity of the enzyme, and promote the immobilized enzyme to have the better activity while the stability and reusability are kept.

## Description

### Technical Field

The application relates to the technical field of biocatalysis, in particular to an enzyme immobilization carrier and a preparation method thereof, an immobilized enzyme and a preparation method thereof.

### Background

Compared with chemical catalysts, biocatalysts, namely enzymes, are widely used in the industrial production process due to its high activity, selectivity and substrate specificity. Biocatalysis becomes an important constituent part of a manufacturing plan for chemicals, intermediates, fine chemicals, and final medicine molecules. Although the enzyme has high regioselectivity, high catalytic efficiency and mild reaction conditions, the catalytic activity of the free enzyme is easily inactivated due to the effects of factors such as temperatures, pH values, and solvents. With the continuous expansion of requirements to medicine process, the requirements for the efficiency and economy of enzyme applications are also higher and higher. Therefore, this not only requires enhancing enzyme activity, specificity, and productivity, but also needs to improve enzyme recoverability and reusability.

A heterogeneous immobilized enzyme system is obtained by immobilizing the enzyme on a solid carrier, which provides a good solution scheme to the above problems. The immobilized enzyme not only maintains the unique catalytic performance of the enzyme, but also shows a series of advantages such as high stability, easy separation and recovery, reusable use, and simple process. The performance of the immobilized enzyme mainly depends on an immobilization method and a carrier material used. According to different binding principle between the enzyme and carrier, the immobilization methods for the enzyme in prior art may be divided into an adsorption method, a covalent binding method, a cross-linking method, and an embedding method and the like. Herein the adsorption method is a combination of physical adsorption, Van Der Waals force and hydrophobic effect and the like between the enzyme and carrier. Because of the advantages of simple operation, large adsorption capacity and low industrial cost and the like, it is the most commonly used method for the immobilized enzyme. However, due to a weaker binding force between the carrier and the enzyme by the physical adsorption, this method has a problem that the enzyme is easily fallen from the carrier during repeated use, so that the conversion rate may be decreased. The covalent binding method is to link the enzyme and the carrier by a covalent bond, and it requires that the carrier must have a corresponding functional group that may react with the enzyme to generate the covalent bond. Compared to the adsorption method, the linkage between the enzyme and the carrier is stronger, there is not the problem that the enzyme is fallen during the use, and the stability and cyclicity are better.

Polyvinyl alcohol (PVA) is a polymer organic compound that is non-toxic, which does not have side effects on a human body, and has good biocompatibility. PVA is widely used in the medical field, it may be made into hydrogel for manufacturing soft contact glasses; it may be made into an oral film for treating bacterial infections after being adhered to an infected part; and it may also be made into a medical sponge for liquid suction and blood suction during a surgery. PVA may be produced by mature methods such as a petroleum ethylene method, a natural gas acetylene method, and a calcium carbide acetylene method, the production is convenient, and the raw material is easy to obtain, and the price is low. In addition, unlike other vinyl polymers (such as polystyrene), PVA may be utilized by bacteria as a carbon source and is degradable, which is an environment-friendly material.

In recent years, a large amount of researches are conducted on bioactive substances immobilized by PVA carriers domestically and internationally, and its preparation methods mainly include a PVA-boric acid method and a freeze-thawing method. The bioactive substances embedded using the PVA-boric acid method is high in mechanical strength, long in service life, and good in elasticity. The disadvantages are that the boric acid has a toxic effect on embedded organisms (enzymes), the residual biological activity is low, and immobilized particles are prone to generate an adhesion phenomenon. The freeze-thawing method forms gel with physical cross-linking, and the gel prepared by this method is relatively large in water content, but the stability of the carrier is not as good as the gel obtained by a chemical cross-linking method, it is very difficult to meet the requirements of some bioreactors, especially a flowing bed reactor. In addition, as an immobilization carrier, in addition to being non-toxic and stable, its mass transfer and loading properties are also important. However, the existing embedding method in the research of immobilized microorganisms is likely to immerse the immobilized microorganisms in chemical reagents or freeze them, so that the activity and application efficiency of the microorganisms may be affected. Moreover, the porosity of an embedding carrier is small, and the mass transfer and loading properties are limited.

Therefore, how to immobilize the enzyme more effectively by PVA, as to make the immobilized enzyme have the higher activity and the better stability and reusability, is an urgent problem to be solved in this field.

### Summary

A main purpose of the application is to provide an enzyme immobilization carrier and a preparation method thereof, an immobilized enzyme and a preparation method thereof, as to solve a problem in existing technologies that a polyvinyl alcohol immobilized enzyme may not have the higher activity and the better stability and reusability.

In order to achieve the above purpose, according to one aspect of the application, an enzyme immobilization carrier is provided, and it is obtained by performing amino modification or cyanuric chloride modification on super-crosslinked polyvinyl alcohol.

Further, the super-crosslinked polyvinyl alcohol is obtained by successively performing oxidizing, self-crosslinking, and crosslinking agent crosslinking on polyvinyl alcohol.

Further, the amino modification uses 3-aminopropyltriethoxysilane as an amino modification reagent.

According to another aspect of the application, an immobilized enzyme is further provided, and it is formed by covalently linking between the above enzyme immobilization carrier and an enzyme.

Further, the enzyme is selected from any one or more of a transaminase, a monooxygenase, a ketoreductase, an ene reductase and an amino acid dehydrogenase; preferably, the transaminase is a transaminase derived from *Chromobacterium violaceum DSM30191,* or a transaminase derived from *Arthrobacter citreus,* or a transaminase derived from *B. thuringiensis;* preferably, the monooxygenase is a cyclohexanone monooxygenase derived from *Brachymonas petroleovorans,* or a cyclohexanone monooxygenase derived from *Rhodococcus ruber-SD1;* preferably, the ketoreductase is a ketoreductase derived from *Acetobacter sp. CCTCC M209061,* or a ketoreductase derived from *Candida macedoniensis AKU4588;* preferably, the ene reductase is an ene reductase derived from *Saccharomyces cerevisiae* and an ene reductase derived from *Chryseobacterium sp. CA49;* and preferably, the amino acid dehydrogenase is a leucine dehydrogenase derived from *Bacillus cereus* and a phenylalanine dehydrogenase derived from *Bacillus sphaericus.*

According to another aspect of the application, a preparation method for the above enzyme immobilization carrier is further provided, and it includes the following steps: super-crosslinked polyvinyl alcohol is provided; and an amino modification or a cyanuric chloride modification is performed on the super-crosslinked polyvinyl alcohol, to obtain the enzyme immobilization carrier.

Further, the super-crosslinked polyvinyl alcohol is prepared by the following method: oxidizing step: polyvinyl alcohol is dissolved in water, and then an oxidant is added for an oxidation reaction, to obtain an oxidation product system; preferably, the oxidant is sodium periodate; preferably, a temperature of the oxidation reaction is 20~30°C; self-crosslinking step: the concentration of hydrogen chloride in the oxidation product system is adjusted to 0.1 ~1 mol/L with hydrochloric acid, and then a self-crosslinking reaction is performed at a temperature of 70~90°C, to obtain polyvinyl alcohol gel particles; crosslinking agent crosslinking step: the polyvinyl alcohol gel particles are dispersed in water, and then a crosslinking agent is added to react, to obtain the super-crosslinked polyvinyl alcohol; preferably, the crosslinking agent is glutaraldehyde solution with a concentration of 2~10wt%; and preferably, the reaction temperature for the crosslinking agent crosslinking step is 40~60°C, and the hydrochloric acid is added during this period so that the hydrogen chloride concentration in the system is 0.1~1 mol/L.

Further, the step of performing the amino modification of the super-crosslinked polyvinyl alcohol includes: the super-crosslinked polyvinyl alcohol, a first solvent, and an amino modification reagent are mixed and reacted, to obtain the enzyme immobilization carrier; preferably, the amino modification reagent is 3-aminopropyltriethoxysilane; preferably, in a reaction process of the amino modification, an addition amount of the amino modification reagent is 0.3~1 mL relative to each gram of the super-crosslinked polyvinyl alcohol; preferably, in the reaction process of the amino modification, a reaction pH value is 2-3, and a reaction temperature is 60~90°C; and preferably, the first solvent is water.

Further, the step of performing the cyanuric chloride modification of the super-crosslinked polyvinyl alcohol includes: the super-crosslinked polyvinyl alcohol is dispersed in a second solvent, and then cyanuric chloride is added to react, to obtain the enzyme immobilization carrier; preferably, an addition amount of the cyanuric chloride is 0.25~1 g relative to each gram of the super-crosslinked polyvinyl alcohol; preferably, in a reaction process of the cyanuric chloride modification, a reaction temperature is 0~10°C; and preferably, the second solvent is acetone.

According to another aspect of the application, a preparation method for an immobilized enzyme is further provided, and it includes the following steps: the above enzyme immobilization carrier and an enzyme are covalently linked, to obtain the immobilized enzyme.

Further, while the enzyme immobilization carrier is obtained by the amino modification of the super-crosslinked polyvinyl alcohol, the preparation method for the immobilized enzyme includes the following steps: the enzyme immobilization carrier is dispersed in glutaraldehyde solution for activation, to obtain an activated carrier; the activated carrier is reacted with enzyme solution containing an enzyme, so that the enzyme is covalently linked with the enzyme immobilization carrier, to obtain the immobilized enzyme; preferably, the mass concentration of the glutaraldehyde solution is 1~2%; more preferably, the glutaraldehyde solution is mixed solution of glutaraldehyde and phosphate buffer; preferably, in the activation step, the activation temperature is 20~30°C, and the activation time is 1-3 h; preferably, in a reaction process of the activated carrier and the enzyme solution, a reaction temperature is 20~30°C; and preferably, each gram of the activated carrier corresponds to 2-6 mL of the enzyme solution, and a protein content in the enzyme solution is 30-40 mg/mL.

Further, while the enzyme immobilization carrier is obtained by the cyanuric chloride modification of the super-crosslinked polyvinyl alcohol, the preparation method for the immobilized enzyme includes the following steps: the enzyme immobilization carrier is wetted with a phosphate buffer; the wetted enzyme immobilization carrier is reacted with the enzyme solution containing an enzyme, so that the enzyme is covalently linked with the enzyme immobilization carrier, to obtain the immobilized enzyme; preferably, in the reaction process of the wetted enzyme immobilization carrier and the enzyme solution, a reaction temperature is 20~30°C; and preferably, each gram of the enzyme immobilization carrier corresponds to 2-6 mL of the enzyme solution, and a protein content in the enzyme solution is 30-40 mg/mL.

The application provides an enzyme immobilization carrier, and it is obtained by the amino modification or the cyanuric chloride modification of the super-crosslinked polyvinyl alcohol. The amino modification or the cyanuric chloride modification is performed on the super-crosslinked polyvinyl alcohol, so that a molecular chain of polyvinyl alcohol may carry an amino group or 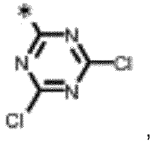 thus these modification groups may be used to react with the enzyme, as to achieve the covalent linkage effect between the two. Therefore, the use of the enzyme immobilization carrier provided by the application may effectively improve the stability and reusability of the immobilized enzyme. Moreover, due to the use of the form of enzyme covalent linkage, compared with an embedding method, there is no need for chemical reagent immersion and the like, it is beneficial to maintain the own activity of the enzyme, and promote the immobilized enzyme to have the better activity while the stability and reusability are kept.

### Detailed Description of the Embodiments

It should be noted that in the case without conflicting, embodiments in the present application and features in the embodiments may be combined with each other. The application is described in detail below in combination with the embodiments.

As described in the background part, the polyvinyl alcohol immobilized enzyme in the existing technologies may not have the higher activity and the better stability and reusability.

In order to solve the above problem, the application provides an enzyme immobilization carrier, and it is obtained by an amino modification or a cyanuric chloride modification of super-crosslinked polyvinyl alcohol. The amino modification or the cyanuric chloride modification is performed on the super-crosslinked polyvinyl alcohol, so that a molecular chain of polyvinyl alcohol may carry an amino group or 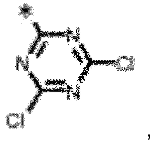 thus these modification groups may be used to react with the enzyme, as to achieve the covalent linkage effect between the two. Therefore, the use of the enzyme immobilization carrier provided by the application may effectively improve the stability and reusability of the immobilized enzyme. Moreover, due to the use of the form of enzyme covalent linkage, compared with an embedding method, there is no need for chemical reagent immersion and the like, it is beneficial to maintain the own activity of the enzyme, and promote the immobilized enzyme to have the better activity while the stability and reusability are kept.

In the application, the super-crosslinked polyvinyl alcohol refers to a highly crosslinked polymer that is insoluble in water and organic solvents and the like, which are good solvents for regular polyvinyl alcohol.

In a preferred implementation mode, the super-crosslinked polyvinyl alcohol is obtained from polyvinyl alcohol by sequentially oxidizing, self-crosslinking, and crosslinking with a crosslinking agent. In the oxidation process, a carbon-carbon bond of an end-to-end connected vicinal diol structure in a PVA chain segment is broken, to form two aldehyde groups; in the self-crosslinking process, the aldehyde group obtained by oxidation may react with a hydroxyl on the PVA chain, to obtain a corresponding acetal structure (oxidizing and self-crosslinking process reactions are as follows). After a glutaraldehyde crosslinking agent is added, it may further react with the hydroxyl on the PVA chain to obtain a super-crosslinked PVA resin.

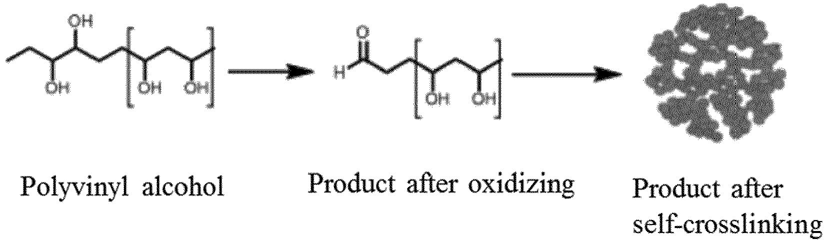

For the purpose of improving the efficiency of the amino modification, in a preferred implementation mode, the amino modification uses 3-aminopropyltriethoxysilane as an amino modification reagent. The 3-aminopropyltriethoxysilane may react with the hydroxyl on the super-crosslinked polyvinyl alcohol molecular chain, to modify a 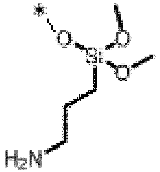 group on the molecular chain. More importantly, this group is selected for the amino modification, the size of the group is more appropriate, and it is gradually changed from rigid into flexible along a direction away from the molecular main chain. It is beneficial to a subsequent immobilization reaction with the enzyme, which is conducive to the subsequent enzyme immobilization reaction, so that the enzyme can be better immobilized, the stability and reusability of the immobilized enzyme are further improved, and it has the better activity.

According to another aspect of the application, an immobilized enzyme is further provided, and it is formed by covalent linkage between the above enzyme immobilization carrier and the enzyme. As described earlier, the enzyme immobilization carrier of the application is formed by performing the amino modification or the cyanuric chloride modification on the super-crosslinked polyvinyl alcohol, so that a molecular chain of polyvinyl alcohol may carry an amino group or 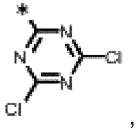 thus these modification groups may be used to react with the enzyme, as to achieve the covalent linkage effect between the two. Therefore, the use of the enzyme immobilization carrier may effectively improve the stability and reusability of the immobilized enzyme. Moreover, due to the use of the form of enzyme covalent linkage, compared with an embedding method, there is no need for chemical reagent immersion and the like, it is beneficial to maintain the own activity of the enzyme, and promote the immobilized enzyme to have the better activity while the stability and reusability are kept.

The immobilized enzyme of the application is widely applicable to enzymes, for example, the enzyme includes but not limited to any one or more of a transaminase, a monooxygenase, a ketoreductase, an ene reductase and an amino acid dehydrogenase. Herein the enzyme is more applicable to the following enzymes, the transaminase is a transaminase derived from *Chromobacterium violaceum DSM30191,* or a transaminase derived from *Arthrobacter citreus,* or a transaminase derived from *B. thuringiensis;* the monooxygenase is a cyclohexanone monooxygenase derived from *Brachymonas petroleovorans,* or a cyclohexanone monooxygenase derived from *Rhodococcus ruber-SD1;* the ketoreductase is a ketoreductase derived from *Acetobacter sp. CCTCC M209061,* or a ketoreductase derived from *Candida macedoniensis AKU4588;* and the amino acid dehydrogenase is a leucine dehydrogenase derived from *Bacillus cereus* and a phenylalanine dehydrogenase derived from *Bacillus sphaericus.*

According to another aspect of the application, a preparation method for the above enzyme immobilization carrier is further provided, and it includes the following steps: super-crosslinked polyvinyl alcohol is provided; and an amino modification or a cyanuric chloride modification is performed on the super-crosslinked polyvinyl alcohol, to obtain the enzyme immobilization carrier. The enzyme immobilization carrier prepared by this method uses the super-crosslinked polyvinyl alcohol as a carrier body, and modifies the amino group or 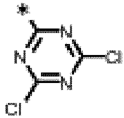 on its molecular chain, these modification groups may react with the enzyme so as to immobilize it, so the immobilized enzyme is formed. Thus, the stability and reusability of the immobilized enzyme may be effectively improved. Moreover, due to the use of the form of enzyme covalent linkage, compared with an embedding method, there is no need for chemical reagent immersion and the like, it is beneficial to maintain the own activity of the enzyme, and promote the immobilized enzyme to have the better activity while the stability and reusability are kept.

In a preferred implementation mode, the super-crosslinked polyvinyl alcohol is prepared by the following method: oxidizing step: polyvinyl alcohol is dissolved in water, and then an oxidant is added for an oxidation reaction, to obtain an oxidation product system; self-crosslinking step: the concentration of hydrogen chloride in the oxidation product system is adjusted to 0.1~1 mol/L with hydrochloric acid, and then a self-crosslinking reaction is performed at a temperature of 70~90°C, to obtain polyvinyl alcohol gel particles; and crosslinking agent crosslinking step: the polyvinyl alcohol gel particles are dispersed in water, and then a crosslinking agent is added to react, to obtain the super-crosslinked polyvinyl alcohol. In the oxidation process, a carbon-carbon bond of an end-to-end connected vicinal diol structure in a PVA chain segment is broken, to form two aldehyde groups. In the self-crosslinking process, the aldehyde group obtained by oxidation may react with a hydroxyl on the PVA chain, to obtain a corresponding acetal structure. After a glutaraldehyde crosslinking agent is added, it may further react with the hydroxyl on the PVA chain to obtain a super-crosslinked PVA resin. Preferably, the oxidant is sodium periodate. The use of the sodium periodate may make an oxidation reaction more complete. Preferably, a temperature of the oxidation reaction is 20~30°C. Preferably, the crosslinking agent is glutaraldehyde; preferably, a reaction temperature for the crosslinking agent crosslinking step is 40~60°C, and the hydrochloric acid is added during this period so that the hydrogen chloride concentration in the system is 0.1 ~1 mol/L. The above crosslinking agent and crosslinking conditions may promote it to more completely react with the hydroxyl on the PVA chain, to form the super-crosslinked polyvinyl alcohol with the more stable structure.

In order to promote dissolution of the polyvinyl alcohol, in the actual operating process, after the first solvent is added to the polyvinyl alcohol, it may be stirred at about 90°C until dissolved. The oxidation process may be performed at a room temperature, and the preferred reaction time is 1~3 h. In the self-crosslinking reaction process, the preferred reaction time is 3~6 h. In the crosslinking agent crosslinking process, the preferred reaction time is 5~8 h.

In a preferred implementation mode, the step of performing the amino modification of the super-crosslinked polyvinyl alcohol includes: the super-crosslinked polyvinyl alcohol, a first solvent, and an amino modification reagent are mixed and reacted, to obtain the enzyme immobilization carrier; preferably, the amino modification reagent is 3-aminopropyltriethoxysilane. The 3-aminopropyltriethoxysilane may react with the hydroxyl on the super-crosslinked polyvinyl alcohol molecular chain, to modify a 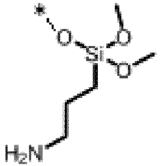 group on the molecular chain. More importantly, this group is selected for the amino modification, the size of the group is more appropriate, and it is gradually changed from rigid into flexible along a direction away from the molecular main chain. It is beneficial to a subsequent immobilization reaction with the enzyme, so that the enzyme is immobilized better, the stability and reusability of the immobilized enzyme are further improved, and it has the better activity.

Preferably, in a reaction process of the amino modification, an addition amount of the amino modification reagent is 1~3 mL relative to each gram of the super-crosslinked polyvinyl alcohol. The addition amount of the amino modification reagent is controlled within the above range, it may more fully react with the hydroxyl on the super-crosslinked polyvinyl alcohol molecular chain, thereby the loading amount and stability of the enzyme may be improved in the subsequent process of immobilizing the enzyme.

For the purpose of further improving reaction efficiency, in a preferred implementation mode, in the reaction process of the amino modification, a reaction pH value is 2-3, and a reaction temperature is 60~90°C. Specifically, a pH adjustor may be used to adjust the pH value of the reaction system, such as a concentrated hydrochloric acid. Preferably, the first solvent is water. Preferably, a reaction time is 5-24 h.

In a preferred implementation mode, the step of performing the cyanuric chloride modification of the super-crosslinked polyvinyl alcohol includes: the super-crosslinked polyvinyl alcohol is dispersed in a second solvent, and then cyanuric chloride is added to react, to obtain the enzyme immobilization carrier. Compared to the amino group as a modification group, it is modified with the cyanuric chloride, the subsequent enzyme immobilization process does not require the use of an additional crosslinking agent such as glutaraldehyde, and the enzyme immobilization carrier may be directly reacted with the enzyme solution. Preferably, an addition amount of the cyanuric chloride is 0.25~1 g relative to each gram of the super-crosslinked polyvinyl alcohol. The amount of the cyanuric chloride is controlled within the above range, it is beneficial to promote the hydroxyl on the molecular chain to more fully react with the cyanuric chloride, thus the enzyme loading amount of the carrier is improved, and the stability and activity of the immobilized enzyme are further improved.

In order to further improve the stability and efficiency of the reaction, in a preferred implementation mode, in the reaction process of the cyanuric chloride modification, the reaction temperature is 0~10°C. Preferably, the second solvent is acetone. Preferably, the reaction time is 2~6 h.

According to another aspect of the application, a preparation method for an immobilized enzyme is further provided, and it includes the following steps: the above enzyme immobilization carrier and an enzyme are covalently linked, to obtain the immobilized enzyme. By the above carrier, an amino or a cyanuric chloride modification group may be used to react with the enzyme, thereby the covalent linkage is formed between the enzyme and the carrier. Compared to physical adsorption immobilization, the immobilized enzyme formed by the covalent linkage has the better stability and reusability. Compared to the embedding method and the like, the use of the covalent linkage promotes the immobilized enzyme to keep the higher enzyme activity, and it has a good improvement effect on long-term stability and maintenance of the catalytic activity.

In a preferred implementation mode, while the enzyme immobilization carrier is obtained by the amino modification of the super-crosslinked polyvinyl alcohol, the preparation method for the immobilized enzyme includes the following steps: the enzyme immobilization carrier is dispersed in glutaraldehyde solution for activation, to obtain an activated carrier; the activated carrier is reacted with enzyme solution containing an enzyme, so that the enzyme is covalently linked with the enzyme immobilization carrier, to obtain the immobilized enzyme. After being activated by the glutaraldehyde, the activated carrier is then reacted with the enzyme solution containing the enzyme, and the modification amino group may be promoted to chemically react with the enzyme, as to achieve the effect of enzyme immobilization. One aldehyde group of the glutaraldehyde reacts with a amino group on the carrier to form an imine bond, and the other aldehyde group reacts with a free amino group on the enzyme, thus the enzyme and the carrier are linked together.

In order to make activation more complete, preferably, the mass concentration of the glutaraldehyde solution is 1~2%; more preferably, the glutaraldehyde solution is mixed solution of glutaraldehyde and phosphate buffer. The above phosphate buffer is preferably a phosphate buffer with pH of 7.0 (it may be a disodium hydrogen phosphate buffer or a sodium dihydrogen phosphate buffer). Preferably, in the activation step, the activation temperature is 20~30°C, and the activation time is 1~3 h. The glutaraldehyde activation is performed at the above temperature, the effect is better, and the reaction is more stable. Preferably, in the reaction process of the activated carrier and the enzyme solution, the reaction temperature is 20~30°C. The enzyme immobilization reaction is performed at the above temperature, and it has the better reaction efficiency and stability. Preferably, each gram of the activated carrier corresponds to 2~6 mL of the enzyme solution, and the protein content in the enzyme solution is 30-40 mg/mL. In this way, it is beneficial to promote the enzyme to be more fully immobilized on the carrier, the enzyme loading amount is improved, and the activity of the immobilized enzyme is improved. Preferably, the reaction time of the above activated carrier and the enzyme solution is 15-25 h.

In a preferred implementation mode, while the enzyme immobilization carrier is obtained by the cyanuric chloride modification of the super-crosslinked polyvinyl alcohol, the preparation method for the immobilized enzyme includes the following steps: the enzyme immobilization carrier is wetted with a phosphate buffer; the wetted enzyme immobilization carrier is reacted with enzyme solution containing an enzyme, so that the enzyme is covalently linked with the enzyme immobilization carrier, to obtain the immobilized enzyme. The above phosphate buffer is preferably a phosphate buffer with pH of 7.0. After being wetted, the cyanuric chloride modification group in the carrier may perform an immobilization reaction with the enzyme in the enzyme solution, as to form covalent linkage between the enzyme and the carrier.

In order to improve the stability in the reaction process and improve the reaction efficiency, in a preferred implementation mode, in the reaction process of the wetted enzyme immobilization carrier and the enzyme solution, the reaction temperature is 20~30°C. More preferably, each gram of the enzyme immobilization carrier corresponds to 2~6 mL of the enzyme solution, and the protein content in the enzyme solution is 30-40 mg/mL. In this way, it is beneficial to promote the enzyme to be more fully immobilized, the loading amount is improved, and the immobilized enzyme has the higher catalytic activity. Preferably, the reaction time of the above enzyme immobilization carrier and the enzyme solution is 15-25 h.

The present application is further described in detail below in combination with specific embodiments, and these embodiments may not be understood as limiting the scope of protection claimed by the present application.

Enzyme sources used in the following embodiments are shown in Table 1 below.

**Table 1**

| Enzyme | Abbreviation | Species source |
|---|---|---|
| Transaminase | TA | Transaminase from *Aspergillus funugatus* |
| Amino acid dehydrogenase | AADH | Leucine Dehydrogenase from *Bacillus cereus* |
| Ketoreductase | KRED | Ketoreductase from *Candida macedoniensis* AKU4588 |
| Monooxygenase | CHMO | Cyclohexanone monooxygensase from *Brachymonas petroleovarans* |
| Ene reductase | ERED | Ene Reductase from *Saocharomyces cerevisiae* |

### Embodiment 1

### Embodiment of immobilization carrier preparation:

PVA oxidizing: PVA (10 g) is added to 100 mL of pure water, and it is stirred at 90°C until dissolved completely. After that, it is cooled to a room temperature, and sodium periodate (0.9 g) is added to it and it is stirred and reacted for 1 h.

PVA self-crosslinking: 36% of a concentrated hydrochloric acid is used to adjust the PVA solution oxidized in the previous step until the hydrogen chloride concentration is 0.1 mol/L, and after that, the temperature is raised to 70°C and it is stilly placed and reacted for 3 hours, to form PVA gel. The self-crosslinking PVA gel obtained is sieved to obtain gel particles (SCL-PVA) (20 meshes), and it is washed for three times with pure water.

Further crosslinking of PVA gel particles: the SCL-PVA gel particles obtained above are dispersed into 100 mL of pure water, and 50wt% of glutaraldehyde (20 mL) and 36% of concentrated hydrochloric acid (25 mL) are added to it, after it is stirred evenly, the temperature is raised to 50°C and it is reacted for 6 hours. After that, the gel particles obtained are washed with the pure water to neutral, and then dried to obtain macroporous super-crosslinked PVA (MP-PVA).

### Embodiment 2

### Embodiment of preparing immobilization carrier by amino modification:

3 g of MP-PVA is taken, 100 mL of deionized water and 2 mL of aminopropyltriethoxysilane (APTES) are added, after it is stirred at a room temperature for 30 min, pH is adjusted to 3.5 with a concentrated hydrochloric acid, after it is stirred for 5 min, the temperature is raised to 80°C, and it is reacted for 20 h under N₂ protection (the reaction route is as follows). After the reaction is completed, it is washed sequentially with ethanol and deionized water, to obtain an amino-modified immobilization carrier MP-PVA-NH₂.

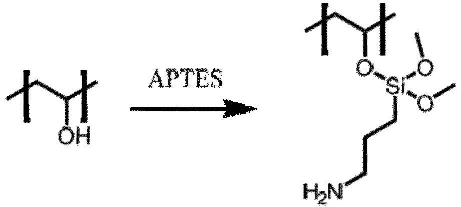

### Embodiment 3

### Embodiment of cyanuric chloride-modified immobilization carrier:

1 g of dried MP-PVA is added to 40 mL of acetone, after it is stirred in an ice-water bath (0°C) for 30 min, 0.5 g of cyanuric chloride is added, and it is continuously reacted in the ice-water bath for 4 h. After the reaction is completed, it is washed with the acetone and suction-filtered until dryness, to obtain a cyanuric chloride-modified enzyme immobilization carrier MP-PVA-CC.

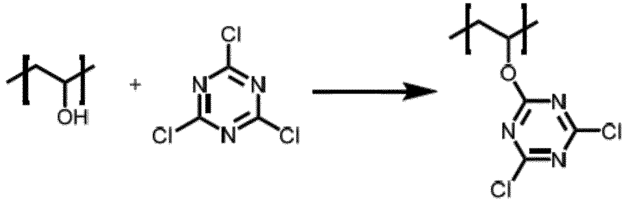

### Embodiment 4

### MP-PVA-NH2 enzyme immobilization:

1wt% of glutaraldehyde solution is prepared with 20 mM of a sodium dihydrogen phosphate buffer with pH of 7.0, and 1 g of MP-PVA-NH2 is weighed and dispersed in the above solution. It is incubated at 30°C for 1 h, then it is washed for three times with deionized water. A certain amount of prepared enzyme solution (the protein content is 30-40 mg/mL) is taken and added to MP-PVA-NH2 activated by glutaraldehyde (4 mL of the enzyme solution corresponds to 1 g of the carrier). It is immobilized at 20°C for 20 h, to prepare an MP-PVA-NH₂ immobilized enzyme.

### Embodiment 5

### MP-PVA-CC enzyme immobilization:

MP-PVA-CC is wetted and washed for three times with 20 mM of a sodium dihydrogen phosphate buffer with pH of 7.0. A certain amount of prepared enzyme solution (the protein content is 30-40 mg/mL) is taken and added into MP-PVA-CC (4 mL of the enzyme solution corresponds to 1 g of the carrier), and it is immobilized at 20°C for 20 h, to prepare an MP-PVA-CC immobilized enzyme.

### Application embodiment 1

MP-PVA-NH₂ and MP-PVA-CC immobilized transaminase (TA) activity and reusability test:
the enzymatic reaction route is as follows.

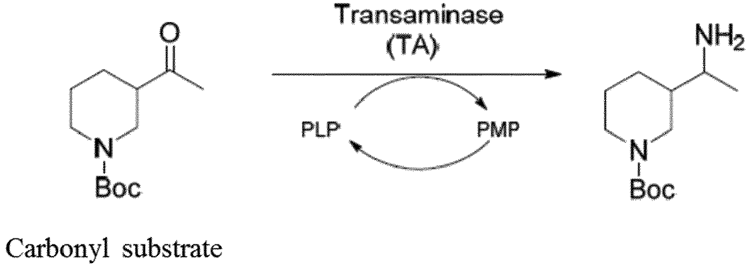

In a 20 mL reaction flask, 0.5 mL of methanol is added to dissolve 0.1 g of the carbonyl substrate, and 15 eq of isopropylamine hydrochloride and 25.0 mg of 5'-pyridoxal phosphate (PLP) are added. 0.1 M of a phosphate buffer (PB 8.0) is supplemented until the final volume of reaction solution is 5 mL, to form a system to be reacted.

0.1 g of transaminase powder or an immobilized enzyme prepared from enzyme solution corresponding to 0.1 g of the transaminase powder are respectively added to the system to be reacted independently, and it is stirred at 47°C for 20 h. The conversion rate of the system is detected by a high performance liquid chromatography (HPLC), and after each round of the reaction, the immobilized enzyme is separated and put into the next round of the reaction for repeated use. The number of repeated uses is investigated. Reaction data is as follows.

| Enzyme | Carrier | Conversion rate (%) | Number of cycles |
|---|---|---|---|
| TA | Free enzyme without a carrier | >97% | 1 |
| | MP-PVA-NH₂ | >90% every cycle | 10 |
| | MP-PVA-CC | >90% every cycle | 10 |

The transaminase is derived Transaminase from *Aspergillus fumigatus* and has the following sequence:
SEQ ID NO:1:

MQKQRTCSQWRELDAAHHLHPFTDTASLNQAGARVMTRGEGVYLWDCEGNKIIDGMAG
LWCVNVGYGRKDFAEAARRQMEELPFYNTFFGTTHPPVVELSSLLAEVTPAGFDRVFYTNSGS
ESVDTMIRMVRRYWDVQGKPEKKTLIGRWNGYHGSTIGGASLGGMKYMHEQGDLPIPGMAHI
EQPWWYKHGKDMTPDEFGVVAARWLEEKILEIGADKVAAFVGEPIQGAGGVIVPPATYWPEIE
RICRKYDVLLVADEVICGFGRTGEWFGHQHFGFQPDLFTAAKGLSSGYLPLGAVFVGDRVAEG
LIAGGDFNHGFTYSGHPVCAAVAHANVAALRDEGIVQRVKDDIGPYMQKRWRETFSRFEHVDD
VRGVGMMLAFTLVKNKAKRELFPDFGEIGTLCEDIFFRNNLIMTAQGDHIVSAPPLVMTRAEVDE
MLAVAERCLEEFEQTLKARGLA.

### Application embodiment 2

MP-PVA-NH₂ and MP-PVA-CC immobilized amino acid dehydrogenase (AADH) activity and reusability test:
the enzymatic reaction route is as follows.

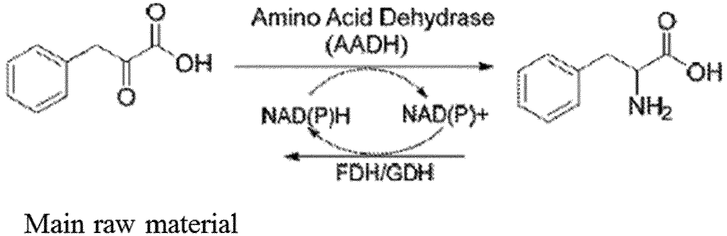

In a 10 mL reaction flask, 5 mL of 0.1 M Tris CI (pH 8.0) is added, then 100 mg of the main raw material and 108 mg of ammonium chloride are added. The pH value is adjusted to 7.5, and then 10 mg of nicotinamide adenine dinucleotide (NAD⁺) and 50 mg of glucose 1-dehydrogenase (GDH: a coenzyme, from *Lysinibacillus sphaericus G10,* is used to recycle nicotinamide adenine dinucleotide phosphate (NADP)) are added to form a system to be reacted.

100 mg of an AADH enzyme (or immobilized AADH prepared from enzyme solution corresponding to 100 mg of the free enzyme) is respectively added to the system to be reacted independently. After it is reacted at 30°C for 20 h, it is used for conversion rate testing. Test results are shown in the table below.

| Enzyme | Carrier | Conversion rate (%) | Number of cycles |
|---|---|---|---|
| AADH | Free enzyme without a carrier | >97% | 1 |
| | MP-PVA-NH₂ | >90% every cycle | 10 |
| | MP-PVA-CC | >90% every cycle | 10 |

The amino acid dehydrogenase is derived from Leucine Dehydrogenase from *Bacillus cereus* and has the following sequence:
SEQ ID NO:2:

MRDVFEMMDRYGHEQVIFCRHPQTGLKAIIALHNTTAGPALGGCRMIPYASTDEALEDVLR
LSKGMTYKCSLADVDFGGGKMVIIGDPKKDKSPELFRVIGRFVGGLNGRFYTGTDMGTNPEDF
VHAARESKSFAGLPKSYGGKGDTSIPTALGVFHGMRATARFLWGTDQLKGRVVAIQGVGKVGE
RLLQLLVEVGAYCKIADIDSVRCEQLKEKYGDKVQLVDVNRIHKESCDIFSPCAKGGVVNDDTID
EFRCLAIVGSANNQLVEDRHGALLQKRSICYAPDYLVNAGGLIQVADELEGFHEERVLAKTEAIY
DMVLDIFHRAKNENITTCEAADRIVMERLKKLTDIRRILLEDPRNSARR.

### Application embodiment 3

MP-PVA-NH₂ and MP-PVA-CC immobilized ketoreductase (KRED) activity and reusability test:
the enzymatic reaction route is as follows.

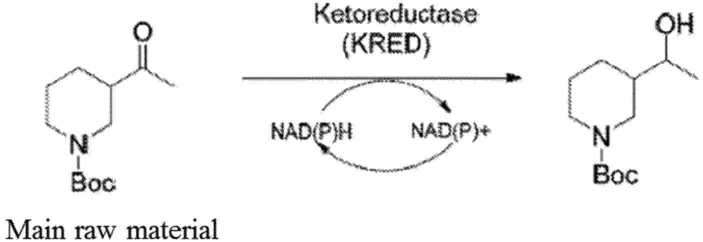

In a 10 mL reaction flask, 0.5 mLof isopropanol (IPA) is added, 0.1 g of the main raw material is dissolved, and 0.5 mL of 0.1 M PB 7.0 and 10 mg of NAD⁺ are added, to form a system to be reacted.

0.05 g of ketoreductase powder or an immobilized enzyme prepared from enzyme solution corresponding to 0.05 g of the ketoreductase powder is respectively added to the system to be reacted independently, and it is stirred at 30°C for 20 h. The conversion rate of the system is detected by HPLC, and after each round of the reaction, the immobilized enzyme is separated and put into the next round of the reaction for repeated use. The number of the repeated uses is investigated. Reaction data is as follows.

| Enzyme | Carrier | Conversion rate (%) | Number of cycles |
|---|---|---|---|
| KRED | Free enzyme without a carrier | >99% | 1 |
| | MP-PVA-NH₂ | >85% every cycle | 10 |
| | MP-PVA-CC | >85% every cycle | 10 |

The ketoreductase is derived from Ketoreductase from *Candida macedoniensis* AKU4588 and has the following sequence.
SEQ ID NO:3:

MKAIQYTRIGAEPELTEIPKPEPGPGEVLLEVTAAGVCHSDDFIMSLPEEQYTYGLPLTLGH
EGAGKVAAVGEGVEGLDIGTNVVVYGPWGCGNCWHCSQGLENYCSRAQELGINPPGLGAPG
ALAEFMIVDSPRHLVPIGDLDPVKTVPLTDAGLTPYHAIKRSLPKLRGGSYAVVIGTGGLGHVAIQ
LLRHLSAATVIALDVSADKLELATKVGAHEVVLSDKDAAENVRKITGSQGAALVLDFVGYQPTID
TAMAVAGVGSDVTIVGIGDGQAHAKVGFFQSPYEASVTVPYWGARNELIELIDLAHAGIFDIAVE
TFSLDNGAEAYRRLAAGTLSGRAVVVPGL.

### Application embodiment 4

MP-PVA-NH₂ and MP-PVA-CC immobilized monooxygenase (CHMO) activity and reusability test:
the enzymatic reaction route is as follows.

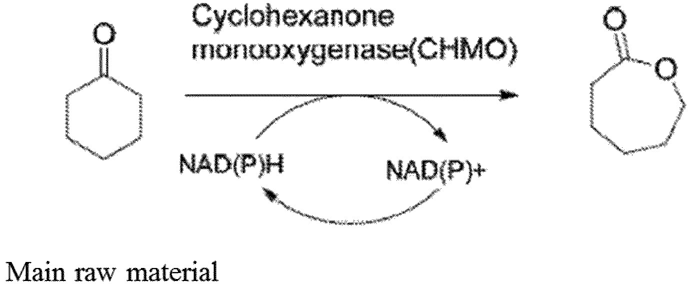

0.3 mL of isopropanol is put into a 10 mL reaction flask, then 500 mg of the main raw material and 3 mL of 0.1 M PB (pH 8.0) containing 5 mg of NADP+ are added, and 50 mg of alcohol dehydrogenase (ADH-Tb: a coenzyme, Alcohol Dehydrogenase from *Thermoanaerobium brockii,* is used to recycle NADP) and 100 mg of monooxygenase enzyme powder (or an immobilized monooxygenase prepared from enzyme solution corresponding to 100 mg of the free enzyme) are added. It is reacted at 30°C for 20 h, the conversion rate is tested, and after each round of the reaction, the immobilized enzyme is separated and put into the next round of the reaction for repeated uses. The number of the repeated uses is investigated. Results are shown in the table below.

| Enzyme | Carrier | Conversion rate (%) | Number of cycles |
|---|---|---|---|
| CHMO | Free enzyme without a carrier | >99% | 1 |
| | MP-PVA-NH₂ | >90% every cycle | 10 |
| | MP-PVA-CC | >85% every cycle | 10 |

The monooxygenase is derived from Cyclohexanone monooxygenase from *Brachymonas petroleovorans* and has the following sequence.
SEQ ID NO:4:

MTTSIDREALRRKYAEERDKRIRPDGNDQYIRLDHVDGWSHDPYMPITPREPKLDHVTFAF
IGGGFSGLVTAARLRESGVESVRIIDKAGDFGGVWYWNRYPGAMCDTAAMVYMPLLEETGYM
PTEKYAHGPEILEHCQRIGKHYDLYDDALFHTEVTDLVWQEHDQRWRISTNRGDHFTAQFVGM
GTGPLHVAQLPGIPGIESFRGKSFHTSRWDYDYTGGDALGAPMDKLADKRVAVIGTGATAVQC
VPELAKYCRELYVVQRTPSAVDERGNHPIDEKWFAQIATPGWQKRWLDSFTAIWDGVLTDPSE
LAIEHEDLVQDGWTALGQRMRAAVGSVPIEQYSPENVQRALEEADDEQMERIRARVDEIVTDP
ATAAQLKAWFRQMCKRPCFHDDYLPAFNRPNTHLVDTGGKGVERITENGVVVAGVEYEVDCIV

YASGFEFLGTGYTDRAGFDPTGRDGVKLSEHWAQGTRTLHGMHTYGFPNLFVLQLMQGAAL
GSNIPHNFVEAARVVAAIVDHVLSTGTSSVETTKEAEQAWVQLLLDHGRPLGNPECTPGYYNN
EGKPAELKDRLNVGYPAGSAAFFRMMDHWLAAGSFDGLTFR.

### Application embodiment 5

### MP-PVA-NH₂ and MP-PVA-CC immobilized ene reductase (ERED) activity and reusability test

The enzymatic reaction route is as follows.

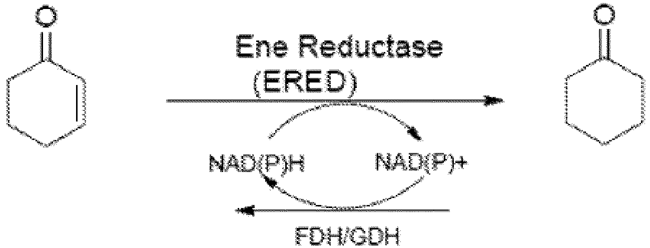

3 mL of 0.1 M sodium dihydrogen phosphate buffer (pH 7.0) is put into a 10 mL reaction flask, and then 100 mg of a substrate is added, and 10 mg of NADP+, 80 mg of ammonium formate and 20 mg of formate dehydrogenase (FDH: a coenzyme, Formate Dehydrogenase from *Candida Boidinii,* is used to recycle NADP) are added, to form a system to be reacted.

100 mg of an ERED enzyme (or an immobilized ERED enzyme prepared from enzyme solution corresponding to 100 mg of the free enzyme) is respectively added to the system to be reacted independently. It is reacted at 30°C for 20 h, the conversion rate is tested, and after each round of the reaction, the immobilized enzyme is separated and put into the next round of the reaction for repeated uses. The number of the repeated uses is investigated. Test results are shown in the table below.

| Enzyme | Carrier | Conversion rate (%) | Number of cycles |
|---|---|---|---|
| ERED | Free enzyme without a carrier | >99% | 1 |
| | MP-PVA-NH₂ | >90% every cycle | 10 |
| | MP-PVA-CC | >98% every cycle | 10 |

The ene reductase is derived from Ene Reductase from *Saccharomyces cerevisiae* and has the following sequence.
SEQ ID NO:5:

MNTMLFSPYTIRGLTLKNRIVMSPMCMYSCDTKDGAVRTWHKIHYPARAVGQVGLIIVEAT
GVTPQGRISERDLGIWSDDHIAGLRELVGLVKEHGAAIGIQLAHAGRKSQVPGEIIAPSAVPFDD
SSPTPKEMTKADIEETVQAFQNGARRAKEAGFDVIEIHAAHGYLINEFLSPLSNRRQDEYGGSP
ENRYRFLGEVIDAVREVWDGPLFVRISASDYHPDGLTAKDYVPYAKRMKEQGVDLVDVSSGAIV
PARMNVYPGYQVPFAELIRREADIPTGAVGLITSGWQAEEILQNGRADLVFLGRELLRNPYWPY
AAARELGAKISAPVQYERGWRF.

The above are only preferred embodiments of the application and are not intended to limit the application. For those skilled in the art, the application may have various variations and changes. Any modifications, equivalent replacements, improvements and the like made within the spirit and principles of the application shall be included within the scope of protection of the application.

## Claims

1. An enzyme immobilization carrier, wherein the enzyme immobilization carrier is obtained by performing an amino modification or a cyanuric chloride modification on super-crosslinked polyvinyl alcohol.

2. The enzyme immobilization carrier according to claim 1, wherein the super-crosslinked polyvinyl alcohol is obtained by successively performing oxidizing, self-crosslinking and crosslinking agent crosslinking on polyvinyl alcohol.

3. The enzyme immobilization carrier according to claim 1 or 2, wherein the amino modification uses 3-aminopropyltriethoxysilane as an amino modification reagent.

4. An immobilized enzyme, wherein the immobilized enzyme is formed by covalently linking the enzyme immobilization carrier according to any one of claims 1 to 3 and an enzyme.

5. The immobilized enzyme according to claim 4, wherein the enzyme is selected from any one or more of a transaminase, a monooxygenase, a ketoreductase, an ene reductase, and an amino acid dehydrogenase;
the transaminase is a transaminase derived from *Chromobacterium violaceum* DSM30191, or a transaminase derived from Arthrobacter citreus, or a transaminase derived from B. *thuringiensis;*
the monooxygenase is a cyclohexanone monooxygenase derived from Brachymonas petroleovorans, or a cyclohexanone monooxygenase derived from *Rhodococcus ruber-SD1;*
the ketoreductase is a ketoreductase derived from *Acetobacter sp.* CCTCC M209061, or a ketoreductase derived from *Candida macedoniensis* AKU4588;
the ene reductase is an ene reductase derived from *Saccharomyces cerevisiae* and an enoly reductase derived from *Chryseobacterium sp.* CA49; and
the amino acid dehydrogenase is a leucine dehydrogenase derived from *Bacillus cereus* and a phenylalanine dehydrogenase derived from *Bacillus sphaericus.*

6. A preparation method for the enzyme immobilization carrier according to any one of claims 1 to 3, wherein the preparation method comprises the following operations:
providing super-crosslinked polyvinyl alcohol; and
performing an amino modification or a cyanuric chloride modification on the super-crosslinked polyvinyl alcohol, to obtain the enzyme immobilization carrier.

7. The preparation method for the enzyme immobilization carrier according to claim 6, wherein the super-crosslinked polyvinyl alcohol is prepared by the following methods:
oxidizing operation: dissolving polyvinyl alcohol in water, and then adding an oxidant to perform an oxidation reaction, to obtain an oxidation product system;
self-crosslinking operation: adjusting the hydrogen chloride concentration of the oxidation product system to 0.1-1 mol/L by using hydrochloric acid, and then performing a self-crosslinking reaction at a temperature of 70-90°C, to obtain polyvinyl alcohol gel particles; and
crosslinking agent crosslinking operation: dispersing the polyvinyl alcohol gel particles in water, and then adding a crosslinking agent to react, to obtain the super-crosslinked polyvinyl alcohol.

8. The preparation method for the enzyme immobilization carrier according to claim 7, wherein the oxidant is sodium periodate; a temperature of the oxidation reaction is 20-30°C.

9. The preparation method for the enzyme immobilization carrier according to claim 7, wherein the crosslinking agent is glutaraldehyde solution of which the concentration is 2-10wt%; a reaction temperature of the crosslinking agent crosslinking operation is 40-60°C, and hydrochloric acid is added during this period to make the hydrogen chloride concentration in the system to be 0.1-1 mol/L.

10. The preparation method for the enzyme immobilization carrier according to any one of claims 6 to 9, wherein the operation of performing the amino modification on the super-crosslinked polyvinyl alcohol comprises:
mixing the super-crosslinked polyvinyl alcohol, a first solvent, and an amino modification reagent and reacting, to obtain the enzyme immobilization carrier.

11. The preparation method for the enzyme immobilization carrier according to claim 10, wherein the amino modification reagent is 3-aminopropyltriethoxysilane;
in a reaction process of the amino modification, an addition amount of the amino modification reagent is 0.3-1 mL relative to each gram of the super-crosslinked polyvinyl alcohol.

12. The preparation method for the enzyme immobilization carrier according to claim 11, whereinin the reaction process of the amino modification, a reaction pH value is 2-3, and a reaction temperature is 60-90°C.

13. The preparation method for the enzyme immobilization carrier according to any one of claims 6 to 9, wherein the operation of performing the cyanuric chloride modification on the super-crosslinked polyvinyl alcohol comprises:
dispersing the super-crosslinked polyvinyl alcohol in a second solvent, and then adding cyanuric chloride to react, to obtain the enzyme immobilization carrier.

14. The preparation method for the enzyme immobilization carrier according to claim 13, wherein an addition amount of the cyanuric chloride is 0.25-1 g relative to each gram of the super-crosslinked polyvinyl alcohol.

15. The preparation method for the enzyme immobilization carrier according to claim 13, wherein in a reaction process of the cyanuric chloride modification, a reaction temperature is 0-10°C.

16. A preparation method for the immobilized enzyme according to claim 4 or 5, wherein the preparation method comprises the following operations: covalently linking the enzyme immobilization carrier according to any one of claims 1 to 3 and an enzyme, to obtain the immobilized enzyme.

17. The preparation method for the immobilized enzyme according to claim 16, wherein while the enzyme immobilization carrier is obtained by performing the amino modification on the super-crosslinked polyvinyl alcohol, the preparation method for the immobilized enzyme comprises the following operations:
dispersing the enzyme immobilization carrier in glutaraldehyde solution and activating, to obtain an activated carrier;
enabling the activated carrier to react with enzyme solution containing an enzyme, so that the enzyme and the enzyme immobilization carrier are covalently linked, to obtain the immobilized enzyme.

18. The preparation method for the enzyme immobilization carrier according to claim 17, the mass concentration of the glutaraldehyde solution is 1-2%.

19. The preparation method for the enzyme immobilization carrier according to claim 18,in the activating operation, an activation temperature is 20-30°C, and activation time is 1-3 h;
in a reaction process of the activated carrier and the enzyme solution, a reaction temperature is 20-30°C; and
each gram of the activated carrier corresponds to 2-6 mL of the enzyme solution, and a protein content in the enzyme solution is 30-40 mg/mL.

20. The preparation method for the immobilized enzyme according to claim 16, wherein while the enzyme immobilization carrier is obtained by performing the cyanuric chloride modification on the super-crosslinked polyvinyl alcohol, the preparation method for the immobilized enzyme comprises the following operations:
wetting the enzyme immobilization carrier by using phosphate buffer solution;
enabling the wetted enzyme immobilization carrier to react with the enzyme solution containing an enzyme, so that the enzyme and the enzyme immobilization carrier are covalently linked, to obtain the immobilized enzyme.

21. The preparation method for the immobilized enzyme according to claim 20, in a reaction process of the wetted enzyme immobilization carrier and the enzyme solution, a reaction temperature is 20-30°C; and
each gram of the enzyme immobilization carrier corresponds to 2-6 mL of the enzyme solution, and a protein content in the enzyme solution is 30-40 mg/mL.
